# EUROPEAN PATENT SPECIFICATION

(11) **EP 3 270 898 B1**
(45) Date of publication and mention of the grant of the patent: **05.05.2021**
(21) Application number: 16765759.2
(22) Date of filing: 17.03.2016
(51) Int. Cl.: A61K 9/127, A01N 57/00, A61P 35/00

(54) **METHODS FOR THE TREATMENT OF BLADDER CANCER**
VERFAHREN ZUR BEHANDLUNG VON BLASENKREBS
PROCÉDÉS DE TRAITEMENT DE CANCER DE LA VESSIE

(30) Priority: 17.03.2015 US 201562134328 P
(43) Date of publication of application: 24.01.2018
(73) Proprietor: Lipomedix Pharmaceuticals Ltd., 9139102 Jerusalem (IL)
(72) Inventor: GABIZON, Alberto, Jerusalem 9640804 (IL); OHANA, Patricia, Jerusalem 96920 (IL)
(74) Representative: Gibson, Mark
(86) International application number: PCT/US2016/022909
(87) International publication number: WO 2016/149516

(56) References cited:
- WO-A1-2015/191563
- US-A1- 2004 161 455
- US-A1- 2005 165 227
- LI ET AL: "Therapeutic effect of Folate Targeted and PEGylated Phytosomes Loaded with Mitomycin C Soybean Phospatidylcholine Complex", MOL. PHARMACEUTICS, vol. 11, no. 9, 2014, pages 3017-3026, XP002784601, DOI: 10.1021/mp5001873
- JEONG ET AL.: 'Comparison of 30 mg and 40 mg of Mitomycin C Intravesical Instillation in Korean Superficial Bladder Cancer Patients: Prospective' RANDOMIZED STUDY. CANCER RESEARCH AND TREATMENT vol. 37, no. 1, 2005, pages 44 - 47, XP055311418
- PATIL ET AL.: 'Targeting of pegylated liposomal mitomycin-C prodrug to the folate receptor of cancer cells: Intracellular activation and enhanced cytotoxicity.' JOURNAL OF CONTROLLED RELEASE vol. 225, 22 January 2016, pages 87 - 95, XP029438893

## Description

### TECHNICAL FIELD

The subject matter described herein relates to compositions for use in treating bladder cancer by intravesical instillation of a liposomal prodrug of mitomycin C.

### BACKGROUND

Neoplasms of the bladder generally originate as pre-malignant lesions and can develop into invasive cancer. A common bladder neoplasm is a transitional cell carcinoma of epithelial origin. Patients with superficial bladder malignancy have a good prognosis but deep invasion of the underlying musculature reduces five year survival to about 50%.

Surgery is a treatment method. The extent of surgery is dependent on the pathological stage of the disease. Early, non-invasive, disease is generally treated by transurethral resection of bladder tumors and intravesical chemotherapy. Trans-urethral surgery is often combined with adjuvant intravesical instillation of chemotherapeutic or immunotherapeutic agents to reduce the incidence and severity of recurrence of cancer either at the same site or at another site on the bladder wall. For superficial cancer, chemotherapy is applied intravesically (directly into the bladder) to concentrate the drug at the tumor site, and eliminate any residual tumor after resection. Muscle-invasive disease is usually managed by radical cystectomy and urinary diversion with or without adjuvant (post-surgery) or neo-adjuvant (pre-surgery) systemic chemotherapy. Definitive (curative) radiotherapy is generally reserved for bladder cancer patients who are not candidates for surgery. Systemic chemotherapy can also be used to manage advanced (metastatic) bladder cancer.

Cancer of the urinary bladder is staged based on the degree of invasion with superficial tumors being the lowest score, while muscle invasion and spread to adjacent structures are more serious. In more advanced stages, the tumor may invade regional lymph nodes or metastasize to distant organs. Cancer cell types also vary from carcinomas to leiomyosarcomas to small cell tumors. Mortality statistics worsen with the depth of invasion. Morbidity varies with both the depth of invasion and the type of treatment given.

There is a high rate of recurrence with bladder cancer. A majority of newly diagnosed bladder tumors are confined to the mucosa (stage Ta, *in situ* tumor) or submucosa (stage T1) and are amenable to transurethral surgical resection. However, after tumor resection recurrence is high, with some estimates of about 70% recurrence rate. This has led to widespread use of local chemotherapy or immunotherapy to reduce risk of recurrence. Attempts to decrease this high recurrence rate include immediate postoperative intravesical instillation of chemotherapy or immunotherapy given a few weeks after tumor resection. When bladder tumor recurs despite adjuvant local therapy, the conservative therapeutic options are limited.
Li et al (2014) Mol. Pharmaceutics, 11(9), 3017-3026 describes the therapeutic effect of folate targeted and PEGylated phytosomes loaded with mitomycin C soybean.

The foregoing examples of the related art and limitations related therewith are intended to be illustrative and not exclusive. Other limitations of the related art will become apparent to those of skill in the art upon a reading of the specification and a study of the drawings.

### BRIEF SUMMARY

The following aspects and embodiments thereof described and illustrated below are meant to be exemplary and illustrative, not limiting in scope.

In one aspect, there is provided a composition comprising liposomes comprising a folate targeting moiety and a prodrug of mitomycin C for use in the treatment of bladder cancer, wherein the prodrug of mitomycin C is a conjugate of mitomycin C releasably attached to a lipophilic moiety, and is of the form: 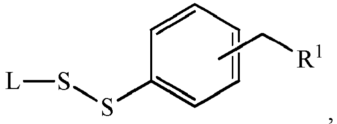 wherein L is the hydrophobic moiety, R¹ represents a mitomycin C residue, and orientation of the -CH₂R¹ group is selected from the ortho position and the para position.

In another embodiment, the liposomes comprising a folate targeting moiety and a prodrug of mitomycin C is administered via intravesical instillation into the bladder. In another embodiment, the liposomes comprising a folate targeting moiety and a prodrug of mitomycin C is in a composition that is administered intravesically. The composition, in one embodiment of the disclosure, comprises a polymer.

In another embodiment of the disclosure, the liposomes or the composition comprising the liposomes is retained in the bladder for a period of between about 15 minutes and about 4 hours. In another embodiment, the liposomes or the composition comprising the liposomes is retained in the bladder for a period of between about 15 minutes and about 2 hours.

In other embodiments, the composition for use is utilized in an individual with Stage 0 bladder cancer or with Stage I bladder cancer. In another embodiment, the composition for use is utilized in an individual low-grade or high-grade bladder cancer.

In addition to the exemplary aspects and embodiments described above, further aspects and embodiments will become apparent by reference to the drawings and by study of the following descriptions.

Additional embodiments of the present composition for use will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded from any embodiment of the present invention. Additional aspects and advantages of the present invention are set forth in the following description and claims, particularly when considered in conjunction with the accompanying examples and drawings.

### BRIEF DESCRIPTION OF THE DRAWINGS

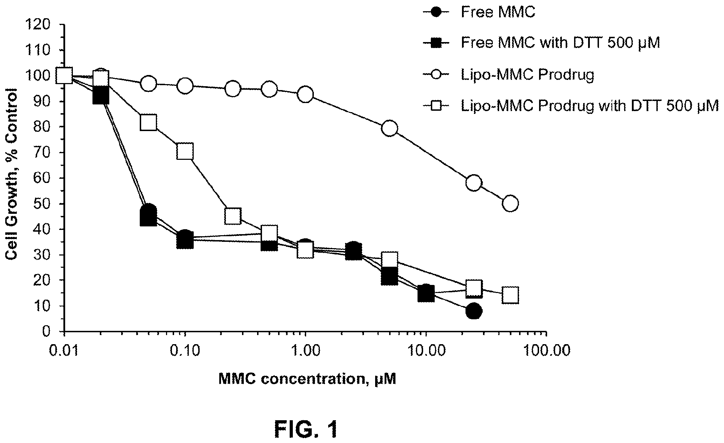

**FIG. 1** is a graph showing growth of T24 human bladder cancer cells, as a percent relative to control (untreated) cells, *in vitro* for cell cultures exposed to various concentration of mitomycin C, in µM, provided in the form of (i) liposomal-mitomycin C prodrug (open circles); (ii) free mitomycin C (closed circles); (iii) liposomal-mitomycin C prodrug and 500 µM dithiothreitol (DTT (open squares); (iv) free mitomycin C and 500 µM dithiothreitol (closed squares);

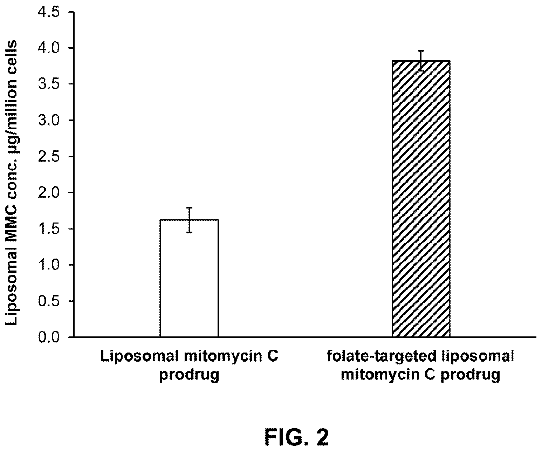

**FIG. 2** is a bar graph showing the uptake into T24 high folate receptors (HiFR) human bladder cancer cells after *in vitro* exposure to liposomal-mitomycin C prodrug (open bar) and to folate-targeted liposomal-mitomycin C prodrug (filled bar); and

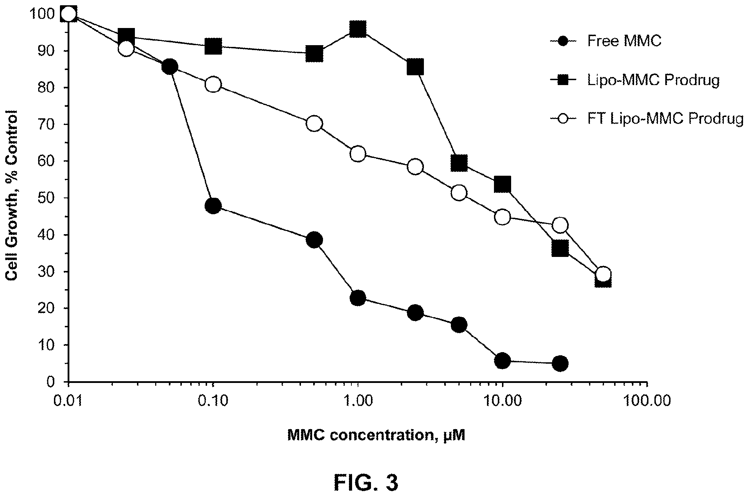

**FIG. 3** is a graph showing growth of T24HiFR human bladder cancer cells, as a percent relative to control (untreated) cells, *in vitro* for cell cultures exposed to various concentration of mitomycin C, in µM, for 24 hours, the mitomycin C provided in the form of (i) liposomal-mitomycin C prodrug (closed squares); (ii) free mitomycin C (closed circles); or (iii) folate-targeted liposomal-mitomycin C prodrug (open circles).

### DETAILED DESCRIPTION

### I. Definitions

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm.

The singular forms "a," "an," and "the" include plural referents unless the context clearly dictates otherwise. Thus, for example, reference to a "liposome" includes a single liposome as well as two or more of the same or different liposomes, reference to an "excipient" includes a single excipient as well as two or more of the same or different excipients, and the like.

As used herein, the term "about" means plus or minus 10% of the numerical value of the number with which it is being used.

As used herein, the term "administration" or "administering" when used in conjunction with a therapeutic means to administer a therapeutic directly into or onto a target tissue or to administer a therapeutic to a patient whereby the therapeutic positively impacts the tissue to which it is targeted. Thus, as used herein, the term "administering", when used in conjunction with a neoplastic agent, can include, but is not limited to, providing a neoplastic agent into or onto the target tissue, or providing a neoplastic agent to a subject by, e.g., intravesical administration.

An amount of liposomal-mitomycin C prodrug that yields a therapeutically-effective amount of mitomycin C after administration is an amount of mitomycin C that is effective to ameliorate or minimize the clinical impairment or symptoms of the neoplasia, in either a single or multiple doses.

As used herein, "prodrug" means a compound that is a drug precursor which, following administration to a subject, releases the drug *in vivo* via some chemical or physiological process such that the prodrug is converted into a product that is toxic to cells of a neoplasm.

A "therapeutically effective amount" or "effective amount" of a composition is a predetermined amount calculated to achieve the desired effect, i.e., to decrease or prevent bladder cancer or the recurrence of bladder cancer. The activity contemplated includes both medical therapeutic and/or prophylactic treatment, as appropriate. The specific dose of a compound administered to obtain therapeutic and/or prophylactic effects will, of course, be determined by the particular circumstances surrounding the case, including, for example, the compound administered, the route of administration, and the condition being treated. However, it will be understood that the effective amount administered will be determined by the physician in the light of the relevant circumstances including the condition to be treated, the choice of compound to be administered, and the chosen route of administration, and therefore the above dosage ranges are not intended to limit the scope in any way. A therapeutically effective amount of compound is typically an amount such that when it is administered in a physiologically tolerable excipient composition, it is sufficient to achieve an effective systemic concentration or local concentration in the tissue.

### II. Compositions for Use

The present disclosure is based in part on the finding that folate-targeted liposomes with a prodrug of mitomycin C are stable in urine for a time sufficient for therapy in the bladder. The composition to be administered is referred to herein as folate-targeted mitomycin C prodrug liposomes. As will be described below, the liposomes in the composition are comprised of a membrane and an intraliposomal core, the membrane comprising at least one liposome forming lipid and a folate targeting moiety exposed at the membrane's outer surface; and mitomycin C prodrug within the liposomal membrane or within the liposomal core. The folate-targeted mitomycin C prodrug liposomes are provided for the treatment of an individual with bladder cancer, where a composition comprising the liposomes is administered in an amount that yields a therapeutically-effective amount of mitomycin C.

### A. Liposomal Mitomycin C Prodrug

The liposomal prodrug conjugate of mitomycin C provided in the compositions for use described herein is comprised of mitomycin C releasably attached to a lipophilic or hydrophobic moiety, and generally is of the form: 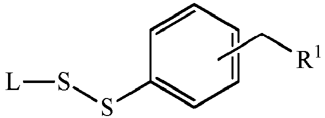 wherein L is a hydrophobic moiety, R¹ represents a mitomycin C residue covalently attached to the dithiobenzyl moiety. Orientation of the CH₂R¹ group is selected from the ortho position and the para position. Synthesis of the conjugate is described in U.S. Patent Nos. 6,365,179; 6,984,396; and 7,303,760.

The hydrophobic moiety, L, is typically a lipid such as a diacylglycerol, a sterol, a phospholipid, or a derivative of these lipids, other naturally-occurring lipids and their synthetic analogs. The hydrophobic moiety is suitable for incorporation into a liposomal bilayer, to anchor the mitomycin C conjugate to a liposomal delivery vehicle.

The liposomal-mitomycin C prodrug conjugate upon exposure to reducing conditions, i.e., a reducing agent such as cysteine or glutathione, decomposes to yield mitomycin C. That is, thiolytic cleavage of the conjugate yields mitomycin C and non-toxic by products of the hydrophobic moiety and the dithiobenzyl moiety. As can be appreciated, the prodrug conjugate can be readily incorporated into liposomes for administration *in vivo* to a subject. The prodrug conjugate is not toxic, and after administration and upon exposure to endogenous reducing agents or exposure to an exogenous reducing agent, the conjugate decomposes to yield mitomycin C in its native state and with biological activity.

Studies conducted in support of the compositions for use described herein used the prodrug conjugate para-diacyldiglyceroldithiobenzal-mitomycin C. The conjugate was synthesized as set forth in Example 1 and was incorporated into a liposomal delivery vehicle, also as described in Example 1.

### B. Folate Targeting Ligand

The liposomes comprise a targeting moiety exposed at least partially at the liposome's outer surface. The targeting moiety may be any ligand that can associate (covalently or non-covalently) to the outer surface of the liposome and have affinity to a target tissue or target organ. Some non-limiting targeting moieties include folate, luteinizing-hormone-releasing hormone (LH-RH); the growth inhibiting hormone, somatostatin, the blood plasma protein, transferrin; target specific antibody such as anti Her2, anti EGFr, anti-nucleosome. The targeting moiety is typically between about 0.05-1.0%, 0.1-0.8%, or 0.1-0.5% in molar ratio of the total lipid content in the liposome.

In the composition for use in treating bladder cancer, a liposome composition comprising a folate targeting moiety is provided. The liposomes comprise a liposome forming lipid (one or more) and a folate conjugate. In one particular embodiment of the disclosure, the molar ratio between the liposome components is, 55:30:10:5:0.5, for HSPC, cholesterol, mPEG-DSPE, and folate ligand conjugate respectively without being limited thereto.

The targeted liposomes may be prepared by any method known to those versed in the art of liposomes. In one embodiment of the disclosure, the targeted liposomes are prepared by using a conjugate between the targeting moiety and a membrane forming component, such as a lipopolymer. The conjugate may be mixed with liposome forming lipids to form the targeted liposome or it may be mixed (incubated) with pre-formed liposomes under conditions which permit the incorporation of the lipopolymer portion of the conjugate into the liposome's membrane. In one embodiment of the disclosure, the conjugate is a conjugate of folate and PEG-lipid, such as folate-PEGsooo-DSPE or folate-PEG₃₃₅₀-DSPE, where the number in subscript denotes the molecular weight of the PEG.

### C. Liposome Lipids, Other Components and Preparation

The liposomes comprise at least one liposome forming lipid, typically at least one phospholipid, forming the liposomes' bilayer membrane which encloses the intraliposomal aqueous phase/core. The term "liposome forming lipids" as used herein denotes those lipids having a glycerol backbone wherein at least one, preferably two, of the hydroxyl groups at the head group is substituted by one or more of an acyl, an alkyl or alkenyl chain, a phosphate group, preferably an acyl chain (to form an acyl or diacyl derivative), a combination of any of the above, and/or derivatives of same, and may contain a chemically reactive group (such as an amine, acid, ester, aldehyde or alcohol) at the headgroup, thereby providing a polar head group. Typically, a substituting chain, e.g. the acyl, alkyl or alkenyl chain, is between about 14 to about 24 carbon atoms in length, and has varying degrees of saturation, thus resulting in fully, partially or non-hydrogenated (liposome-forming) lipids.

Further, the lipids may be of a natural source, semi-synthetic or a fully synthetic lipid, and may be neutral, negatively or positively charged. There are a variety of synthetic vesicle-forming phospholipids and naturally-occurring vesicle-forming phospholipids, including the phospholipids, such as phosphatidylcholine (PC), phosphatidylinositol (PI), phosphatidylglycerol (PG), dimyristoyl phosphatidylglycerol (DMPG); egg yolk phosphatidylcholine (EPC), 1-palmitoyl-2-oleoylphosphatidyl choline (POPC), distearoylphosphatidylcholine (DSPC), dimyristoyl phosphatidylcholine (DMPC); phosphatidic acid (PA), phosphatidylserine (PS); 1-palmitoyl-2-oleoylphosphatidyl choline (POPC), and the sphingophospholipids such as sphingomyelins (SM) having 12- to 24-carbon atom acyl or alkyl chains.

Lipids having a relatively high Tₘ may be referred to as "rigid" lipids, typically those having saturated, long acyl chains, while lipids with a relatively low Tₘ may be referred to as "fluid" lipids. Fluidity or rigidity of the liposome may be determined by selecting lipids with pre-determined fluidity/rigidity for use as the liposome-forming lipids. In accordance with one embodiment of the disclosure, the Tₘ of the lipids forming the liposomes is preferably equal to or above 30 °C or equal to or above 40 °C.

A non-limiting example of lipids forming the liposomes and having a Tₘ above 30 °C comprises phosphatidylcholine (PC) and derivatives thereof having two acyl (or alkyl) chains with 16 or more carbon atoms. Some preferred examples of PC derivatives which form the basis for the low permeable liposomes in the context of the disclosure include, without being limited thereto, hydrogenated soy PC(HSPC) having a Tₘ of 52 °C, Dipalmitoylphosphatidylcholine (DPPC), having a Tₘ of 41.3 °C, N-palmitoyl sphingomyelin having a Tₘ of 41.2 °C, distearylphosphatidylcholine (DSPC) having a Tₘ of 55 °C, N-stearoyl sphingomyelin having a Tₘ of 48 °C; distearyolphosphatidylglycerol (DSPG) having a Tₘ of 55 °C, partially hydrogenated phosphatidylcholine (PHPC) having a Tₘ of 35 °C; and distearyphosphatidylserine (DSPS) having a Tₘ of 68 °C. The above Tₘ temperature data are from http://www.avantilipids.com Phase Transition Temperatures or from http://www.lipidat.tcd.ie., as known to those versed in the art. Those versed in the art will know how to select a lipid with a Tₘ either equal or above 25 °C, 30 °C or equal or above 40 °C see also Barenholz, Y., Curr. Opin. Colloid Interface Sci. 6, 66-77 (2001); Barenholz, Y. and Cevc, G., Structure and properties of membranes. In Physical Chemistry of Biological Surfaces (Baszkin, A. and Norde, W., eds.), Marcel Dekker, NY (2000) pp. 171-2411).

The liposomes may further comprise membrane active sterols (e.g. cholesterol) and/or phosphatidylethanolamines in order to decrease a membrane's free volume and thereby permeability and leakage of material loaded therein. In one embodiment of the disclosure, the membrane comprises cholesterol. The addition of sterol is known to affect permeability of the liposomes.

The liposomes may also include a lipid derivatized with a hydrophilic polymer to form new entities known by the term lipopolymers. Lipopolymers preferably comprise lipids (liposome forming lipids as well as lipids that do not form into lipids, such as phosphatidylethanolamines) modified at their head group with a polymer having a molecular weight equal to or above 750 Daltons (Da). The head group may be polar or apolar; however, it is preferably a polar head group to which a large (>750 Da), highly hydrated (at least 60 molecules of water per head group), flexible polymer is attached. The attachment of the hydrophilic polymer head group to the lipid region may be a covalent or non-covalent attachment; however, it is preferably via the formation of a covalent bond (optionally via a linker). The outermost surface coating of hydrophilic polymer chains is effective to provide a liposome with a long blood circulation lifetime *in vivo.* Examples have been described in Tirosh *et al.* (Tirosh et al., Biopys. J., 74(3): 1371-1379, (1998)) and in U.S. Pat. Nos. 5,013,556; 5,395,619; 5,817,856; 6,043,094; and 6,165,501; and in WO 98/07409. The lipopolymers may be non-ionic lipopolymers (also referred to at times as neutral lipopolymers or uncharged lipopolymers) or lipopolymers having a net negative or a net positive charge.

There are numerous polymers which may be attached to lipids. Polymers typically used as lipid modifiers include, without being limited thereto: polyethylene glycol (PEG), polysialic acid, polylactic acid (also termed polylactide), polyglycolic acid (also termed polyglycolide), polylactic-polyglycolic acid, polyvinyl alcohol, polyvinylpyrrolidone, polymethoxazoline, polyethyloxazoline, polyhydroxyethyloxazoline, polyhydroxypropyloxazoline, polyaspartamide, polyhydroxypropyl methacrylamide, polymethacrylamide, polydimethylacrylamide, polyvinylmethylether, polyhydroxyethyl acrylate, derivatized celluloses such as hydroxymethylcellulose or hydroxyethylcellulose. The polymers may be employed as homopolymers or as block or random copolymers.

The lipopolymer may be introduced into the liposome in two different ways either by: (a) adding the lipopolymer to a lipid mixture, thereby forming the liposome, where the lipopolymer will be incorporated and exposed at the inner and outer leaflets of the liposome bilayer (Uster P. S. et al. FEBBS Letters 386:243 (1996)); or (b) first preparing the liposome and then incorporating the lipopolymers into the external leaflet of the pre-formed liposome either by incubation at a temperature above the Tₘ of the lipopolymer and liposome-forming lipids, or by short-term exposure to microwave irradiation.

While the lipids derivatized into lipopolymers may be neutral, negatively charged, or positively charged, i.e. there is no restriction regarding a specific (or no) charge, the most commonly used and commercially available lipids derivatized into lipopolymers are those based on phosphatidyl ethanolamine (PE), usually, distearylphosphatidylethanolamine (DSPE).

A specific family of lipopolymers which may be employed by the disclosure include monomethylated PEG attached to DSPE (with different lengths of PEG chains, the methylated PEG referred to herein by the abbreviation PEG) in which the PEG polymer is linked to the lipid via a carbamate linkage resulting in a negatively charged lipopolymer. Other lipopolymers are the neutral methyl polyethyleneglycol distearoylglycerol (mPEG-DSG) and the neutral methyl polyethyleneglycol oxycarbonyl-3-amino-1,2-propanediol distearoylester (mPEG-DS) (Garbuzenko O. et al., Langmuir, 21:2560-2568 (2005)). The PEG preferably has a molecular weight of the PEG head group from about 750 Da to about 20,000 Da. More preferably, the molecular weight is from about 750 Da to about 12,000 Da, and it is most preferably between about 1,000 Da to about 5,000 Da. One specific PEG-DSPE employed herein is a PEG moiety with a molecular weight of 2000 Da, designated herein PEG₂₀₀₀-DSPE. In another embodiment of the disclosure, the PEG molecule utilized for attachment of the folate targeting ligand has a molecular weight greater than the molecular weight of the PEG utilized in forming a lipopolymer that is utilized for liposome formation. In this way, the folate targeting ligand is not sterically hindered by the PEG chains surrounding the liposome. In one embodiment of the disclosure, the PEG utilized for attachment of a the folate targeting ligand has a molecular weight of greater than 3350 Daltons, preferably between 3350-10,000 Daltons, and preferably between 3350-6000 Daltons, and the PEG utilized in the form of a lipopolymer component in formation of the liposome has a molecular weight of less than 5000 Daltons, preferably less than 3350 Daltons, and preferably between 750-5000 Daltons or between 500-3500 Daltons.

In liposomes including such derivatized lipids it typically includes between 1-20 mole percent of such a derivatized lipid is included in the liposome formulation.

The liposome may include other constituents. For example, charge-inducing lipids, such as phosphatidylglycerol, such as dipalmitoylphosphatidylglycerol (DPPG, Tₘ of about 41 °C) may also be incorporated into the liposome bilayer to decrease vesicle-vesicle fusion, and to increase interaction with cells. Buffers at a pH suitable to make the liposome surface's pH close to neutral can decrease hydrolysis. Addition of an antioxidant, such as vitamin E, or chelating agents, such as deferoxamine (desferal) or diethylene triamine pentaacetic acid (DTPA), may be used.

The liposomes according to the present disclosure are typically those having a diameter of between about 70 nm to 130 nm, or even between about 80 nm and 110 nm. The liposomes may be unilamellar, bilamellar or even, at times, multilamellar. In one embodiment of the disclosure, the liposomes are small unilamellar vesicles (SUV), although the population of liposomes may include also some liposomes with more than one lamella.

### D. Exemplary Studies in Support of the Composition for Use

In a first study, prodrug conjugate of mitomycin C was prepared and incorporated into liposomes as described in Example 1. The mitomycin-C prodrug conjugate incorporated into liposomes is referred to as "liposomal-mitomycin C prodrug." As mentioned above, liposomes with a folate targeting ligand and the mitomycin C prodrug are referred to as "folate-targeted mitomycin C prodrug liposomes" or "folate-targeted liposomal-mitomycin C prodrug."

Compositions comprising the liposomal-mitomycin C prodrug were studied using T24 human bladder cancer cells. FIG. 1 shows the growth of T24 human bladder cancer cells, as a percent relative to control (untreated) cells, *in vitro* for cell cultures exposed to various concentration of mitomycin C, in µM, provided in the form of (i) liposomal-mitomycin C prodrug (open circles); (ii) free mitomycin C (closed circles); (iii) liposomal-mitomycin C prodrug and 500 µM dithiothreitol (DTT (open squares)); (ii) free mitomycin C and 500 µM dithiothreitol (closed squares). The study shows that the liposomal-mitomycin C prodrug composition slows growth of the cancer cells when the liposomal-mitomycin C prodrug is activated by a reducing agent, such as exogenously added dithiothreitol. It will be appreciated that *in vivo* a reducing agent may be endogeneously present at the cell surface or can be added exogenously. A composition of liposomal-mitomycin C prodrug when activated by a reducing agent is as effective in inhibiting cell growth as free mitomycin C.

In another study, described in Example 3, liposomes with the mitomycin C prodrug were prepared using a radiolabelled lipid (cholesterol). Radiolabelled compositions comprising liposomal-mitomycin C prodrug and comprising folate-targeted liposomal-mitomycin C prodrug were prepared and tested for uptake into T24 (HiFR) human bladder cancer cells. FIG. 2 is a bar graph showing the uptake into human bladder cancer cells after *in vitro* exposure for 3 hours to liposomal-mitomycin C prodrug (open bar) and to folate-targeted liposomal-mitomycin C prodrug (filled bar). The folate-targeted liposomes were taken up by the cells about three-times greater than the liposomes absent a folate-targeting moiety. Accordingly, in one embodiment of the disclosure, a composition comprising folate-targeted liposomal-mitomycin C prodrug is administered to a subject with bladder cancer to achieve a two-fold, or three-fold increase in intracellular mitomycin C concentration relative to a composition of liposomal-mitomycin C prodrug lacking the folate targeting moiety.

Another study, described in Example 4, compared the *in vitro* cell growth of T24 (HiFR) human bladder cancer cells after 24 hours of exposure to free mitomycin C, liposomal-mitomycin C prodrug and folate-targeted liposomal-mitomycin C prodrug at various concentrations of mitomycin C. As seen in FIG. 3, growth of T24 human bladder cancer cells decreases with increasing drug concentration, where the decrease in cell growth is greater for cells exposed to folate-targeted liposomal-mitomycin C prodrug (open circles) compared to cells exposed to liposomal-mitomycin C prodrug (closed squares). Cells exposed to free mitomycin C (closed circles) had the slowest growth.

An *in vivo* study was also conducted and is described in Example 5. In this study, rats were treated by intravesical instillation with liposomal-mitomycin C prodrug; or with folate-targeted liposomal mitomycin C prodrug. As controls other rats were treated with phosphate buffered saline or with mitomycin C. Blood, urine and various organs were collected one hour after instillation of the test (or control) composition, and the concentration of liposomal mitomycin C- prodrug, and of mitomycin C were determined. Results are shown in Tables 1-3.

The data in Table 2 shows that no liposomal mitomycin C-prodrug was detected in the blood after treatment via intravesical instillation with liposomal-mitomycin C prodrug or with folate-targeted liposomal mitomycin C prodrug, indicating there was no leaking of the liposomes from the bladder to the blood circulation. The level of liposomal-mitomycin C prodrug in the bladder tissue (Table 3) was higher in the animals treated with folate-targeted liposomal mitomycin C prodrug as compared to the group treated with liposomal-mitomycin C prodrug. The level of liposomal-mitomycin C prodrug in urine (Table 1) of animals treated with the samples of folate-targeted liposomal mitomycin C prodrug was lower than the level measured in the samples from the animals treated with liposomal-mitomycin C prodrug. These results indicate a higher uptake of folate-targeted liposomal-mitomycin C prodrug in the bladder as compared to liposomal-mitomycin C prodrug. Accordingly, in one embodiment of the disclosure, the composition for use contemplates administering via intravesical instillation a composition comprising folate-targeted liposomal-mitomycin C prodrug for a period of at least about 20 minutes, 30 minutes, 45 minutes, 1 hour, or 2 hours, where the administered composition remains resident in the vesical (e.g., bladder) as evidenced by no measurable amount of mitomycin C-liposomal prodrug in the blood of the subject being treated.

**Table 1: Mitomycin C and Mitomycin C Liposomal Prodrug Concentrations in Urine One hour after intravesical instillation**

| Animal No. | Treatment Composition | MMC Concentration in Urine (µg/mL) | MLP¹ Concentration in Urine (µg/mL) | % of injected dose/total urine |
|---|---|---|---|---|
| 1 | PBS | 0 | 0 | 0 |
| 2 | PBS | 0 | 0 | 0 |
| 3 | MMC | 118.7 | n.a.² | 49.58 |
| 4 | MMC | 164.8 | n.a. | 32.96 |
| 5 | MMC | 177.1 | n.a. | 21.25 |
| 6 | liposomal-mitomycin C prodrug | n.a. | 1265 | 101.2 |
| 7 | liposomal-mitomycin C prodrug | n.a. | 1211 | 72.6 |
| 8 | folate-targeted liposomal-mitomycin C prodrug | n.a. | 47 | 0.32 |
| 9 | folate-targeted liposomal-mitomycin C prodrug | n.a. | 23 | 1.04 |

| | | | | |
|---|---|---|---|---|
| ¹MLP = mitomycin C-liposomal prodrug; ²n.a. = not applicable | | | | |

**Table 2: Mitomycin C and Mitomycin C Liposomal Prodrug Concentrations in Plasma One hour after intravesical instillation**

| Animal No. | Treatment Composition | MMC Concentration in Plasma (µg/mL) | MLP¹ Concentration in Plasma (µg/mL) | % of injected dose/total plasma |
|---|---|---|---|---|
| 1 | PBS | 0 | 0 | 0 |
| 2 | PBS | 0 | 0 | 0 |
| 3 | MMC | 0 | 0 | 0 |
| 4 | MMC | 0.24 | n.a.² | 0.44 |
| 5 | MMC | 0.30 | n.a. | 0.54 |
| 6 | liposomal-mitomycin C prodrug | n.a. | 0 | 0 |
| 7 | liposomal-mitomycin C prodrug | n.a. | 0 | 0 |
| 8 | folate-targeted liposomal-mitomycin C prodrug | n.a. | 0.5 | 0.2 |
| 9 | folate-targeted liposomal-mitomycin C prodrug | n.a. | 0 | 0 |

| | | | | |
|---|---|---|---|---|
| ¹MLP = mitomycin C-liposomal prodrug; ²n.a. = not applicable | | | | |

**Table 3: Mitomycin C and Mitomycin C Liposomal Prodrug Concentrations in Bladder Tissue One hour after intravesical instillation**

| Animal No. | Treatment Composition | MMC Concentration in Bladder Tissue (µg/g) | MLP¹ Concentration in Bladder Tissue (µg/g) | % of injected dose/g bladder tissue |
|---|---|---|---|---|
| 1 | PBS | not measured | not measured | not measured |
| 2 | PBS | not measured | not measured | not measured |
| 3 | MMC | 43.0 | n.a.² | 8.6 |
| 4 | MMC | 68.3 | n.a. | 13.7 |
| 5 | MMC | 18.6 | n.a. | 3.7 |
| 6 | liposomal-mitomycin C prodrug | n.a. | 134.49 | 5.38 |
| 7 | liposomal-mitomycin C prodrug | n.a. | 18.15 | 0.73 |
| 8 | folate-targeted liposomal-mitomycin C prodrug | n.a. | 709.79 | 28.39 |
| 9 | folate-targeted liposomal-mitomycin C prodrug | n.a. | 770.39 | 62.35 |

| | | | | |
|---|---|---|---|---|
| ¹MLP = mitomycin C-liposomal prodrug; ²n.a. = not applicable | | | | |

From the foregoing, it will be appreciated that a composition for use in treating bladder cancer is provided, where liposomes comprising a folate targeting moiety and a prodrug of mitomycin C is administered to the individual. The liposomes are administered in an amount that yields a therapeutically-effective amount of mitomycin C for the treatment of bladder cancer. The prodrug is a conjugate of mitomycin C releasably attached to a lipophilic moiety, such that the mitomycin C is released from the liposomes after administration *in vivo.* The *in vivo* study demonstrated that the active agent, the liposomal prodrug of mitomycin C, remains resident in the bladder, with substantially no uptake of the agent into the blood circulation.

In one embodiment, the liposomes comprising a folate targeting moiety and a prodrug of mitomycin C are administered via intravesical instillation into the bladder. The administered liposomes may be retained in the bladder for a period of between about 15 minutes to about 4 hours, or between about 15 minutes and about 2 hours.

The liposomes comprising a folate targeting moiety and a prodrug of mitomycin C can be administered in a simple buffer solution or can be in a composition with pharmaceutically-acceptable excipients, such as polymers or thickening agents. In one embodiment of the disclosure, the composition comprises folate-targeted liposomal mitomycin C and a polymeric material that increases retention of the liposomes in the bladder.

The composition for use is contemplated for treating an individual with any stage or grade of bladder cancer, and in preferred embodiments is intended for treatment of an individual with Stage 0 bladder cancer or Stage I bladder cancer, and with low-grade or high-grade bladder cancer. Stage 0 bladder cancer includes non-invasive papillary carcinoma (Ta) and flat non-invasive carcinoma (Tis). In either case, the cancer has not invaded the bladder wall beyond the inner layer. Stage I bladder cancers have grown into the connective tissue layer of the bladder wall but have not reached the muscle layer.

The actual dosage employed may be varied depending upon the requirements of the patient and the severity of the bladder cancer. Determination of the proper dosage for a particular situation is within the skill of the art. Also, in accordance with the knowledge of the skilled clinician, the therapeutic protocols (e.g., dosage amounts and times of administration) can be varied in view of the observed effects of the administered therapeutic agents (i.e., antineoplastic agent(s) or radiation) on the patient, and in view of the observed responses of the disease to the administered therapeutic agents.

### I. EXAMPLES

The following examples are illustrative in nature and are in no way intended to be limiting.

### EXAMPLE 1

Folate-Targeted Mitomycin C Prodrug Liposome Preparation

### A. Preparation of Liposomal-Mitomycin C Prodrug

A prodrug conjugate of mitomycin C releasably attached to a lipophilic moiety, para-diacyldiglyceroldithiobenzyl-mitomycin C, was synthesized as described in U.S. Patent No. 7,303,760, in Example 2.

The para-diacyldiglyceroldithiobenzyl-mitomycin C prodrug conjugate was incorporated into liposomes as described in Example 3A of U.S. Patent No. 7,303,760. Liposomes with the para-diacyldiglyceroldithiobenzyl-mitomycin C prodrug conjugate are referred to as "liposomal-mitomycin C prodrug."

### B. Preparation of Folate-Targeted Liposomal-Mitomycin C Prodrug

Folate-derivatized PEG₅₀₀₀-DSPE was synthesized as described by Gabizon *et al.* (Gabizon, A. et al. Bioconjugate Chemistry, 10(2):289-98, 1999). The folate-lipophilic conjugate (folate-PEG₅₀₀₀-DSPE, MW=4051 Da) was added to the liposomal-mitomycin C prodrug at 0.5 molar ratio relative to the total phospholipid content. The folate-PEG₅₀₀₀-DSPE was weighed in dry form, suspended in the liposomal-mitomycin C prodrug containing buffer and incubated at 45 °C for 2 hours with shaking for incorporation of the folate-PEG₅₀₀₀-DSPE into the lipid bilayer of the liposomal-mitomycin C prodrug. Then, the liposome suspension was cooled and centrifuged (10 min. at 3000 rpm) to remove any precipitate of non-incorporated folate-PEG₅₀₀₀-DSPE.

Folate-PEG₅₀₀₀-DSPE liposome content was determined spectrophotometrically at 284 nm after disruption of the liposomes by dilution 1:10 in 3% sodium dodecyl sulfate (SDS) as described previously (Gabizon *et al.,* 1999, *ibid*)*.*

### EXAMPLE 2

### In Vitro Cytotoxicity of Liposomal-Mitomycin C Prodrug

A composition comprising liposomal-mitomycin C prodrug was prepared as described in Example 1A. T24 human bladder cancer cells were obtained and cultured. Growth of the cells in the presence of the following compositions was tested *in vitro*: (i) liposomal-mitomycin C prodrug; (ii) free mitomycin C; (iii) liposomal-mitomycin C prodrug and 500 µM dithiothreitol (DTT); (ii) free mitomycin C and 500 µM dithiothreitol (DTT). Results are shown in FIG. 1.

### EXAMPLE 3

### In Vitro Uptake of Folate-Targeted Liposomal-Mitomycin C Prodrug

Compositions comprising liposomal-mitomycin C prodrug and folate-targeted liposomal mitomycin C prodrug, both with radiolabeled cholesterol, were prepared as described in Examples 1A-1B. T24 (HiFR) human bladder cancer cells were obtained and cultured. Uptake of the compositions into human bladder cancer cells after 3 hours exposure to each composition was measured. Results are shown in FIG. 2.

### EXAMPLE 4

### In Vitro Cytotoxicity of Folate-Targeted Liposomal-Mitomycin C Prodrug

A composition comprising liposomal-mitomycin C prodrug was prepared as described in Example 1A. A composition comprising folate-targeted liposomal mitomycin C prodrug was prepared as described in Example 1B.

T24 (HiFR) human bladder cancer cells were obtained and cultured. Growth of the cells in the presence of the following compositions was tested *in vitro*: (i) liposomal-mitomycin C prodrug; (ii) free mitomycin C; and (iii) folate-targeted liposomal-mitomycin C prodrug. Results are shown in FIG. 3.

### EXAMPLE 5

### In Vivo Administration of Liposomal-Mitomycin C Prodrug and Folate-Targeted Liposomal-Mitomycin C Prodrug

A composition comprising liposomal-mitomycin C prodrug was prepared as described in Example 1A. A composition comprising folate-targeted liposomal mitomycin C prodrug was prepared as described in Example 1B.

Nine healthy rats were divided in four test groups for treatment by intravesical instillation with one of the following: Group 1: phosphate buffered saline (PBS); Group 2: free mitomycin C (MMC); Group 3: liposomal-mitomycin C prodrug; and Group 4: folate-targeted liposomal mitomycin C prodrug. Blood, urine and various organs were collected one hour after instillation of the test (or control) composition, and the concentration of mitomycin C liposomal prodrug and of mitomycin C were determined. Results are shown in Tables 1-3.

## Claims

1. A composition comprising liposomes comprising a folate targeting moiety and a prodrug of mitomycin C for use in the treatment of bladder cancer, wherein the prodrug of mitomycin C is a conjugate of mitomycin C releasably attached to a lipophilic moiety, and is of the form: 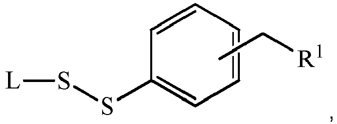 wherein L is the hydrophobic moiety, R¹ represents a mitomycin C residue, and orientation of the -CH₂R¹ group is selected from the ortho position and the para position.

2. The composition for use of claim 1, wherein the composition is formulated for administration via intravesical instillation.

3. The composition for use of claim 1 or claim 2, wherein the composition is formulated for retaining the liposomes in the bladder for a period of between about 15 minutes and about 2 hours.

4. The composition for use of any one of claims 1-3, wherein the composition is formulated for administration with a reducing agent to initiate release of the prodrug conjugate of mitomycin C from the attached lipophilic moiety.

5. The composition for use of any one of claims 1-4, wherein the bladder cancer is Stage 0 bladder cancer or Stage 1 bladder cancer.

6. The composition for use of any one of claims 1-5, wherein the bladder cancer is low-grade or high-grade bladder cancer.

## Patentansprüche

1. Zusammensetzung, die Liposome umfasst, die eine Folat-Targeting-Einheit und ein Prodrug von Mitomycin C umfassen, zur Verwendung bei der Behandlung von Blasenkrebs, wobei das Prodrug von Mitomycin C ein Konjugat von Mitomycin C ist, das lösbar an eine lipophile Einheit gebunden ist, und von der folgenden Form ist: 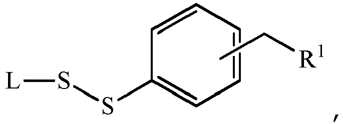 wobei L die hydrophobe Einheit ist, R¹ einen Mitomycin-C-Rest darstellt, und Ausrichtung der Gruppe -CH₂R¹ aus der ortho-Position und der para-Position ausgewählt ist.

2. Zusammensetzung zur Verwendung nach Anspruch 1, wobei die Zusammensetzung zur Verabreichung über intravesikale Instillation formuliert ist.

3. Zusammensetzung zur Verwendung nach Anspruch 1 oder Anspruch 2, wobei die Zusammensetzung zum Halten der Liposome in der Blase für einen Zeitraum zwischen etwa 15 Minuten und etwa 2 Stunden formuliert ist.

4. Zusammensetzung zur Verwendung nach einem der Ansprüche 1-3, wobei die Zusammensetzung zur Verabreichung mit einem Reduktionsmittel formuliert ist, um die Freisetzung des Prodrug-Konjugats von Mitomycin C aus der gebundenen lipophilen Einheit zu initiieren.

5. Zusammensetzung zur Verwendung nach einem der Ansprüche 1-4, wobei der Blasenkrebs im Stadium 0 oder Blasenkrebs im Stadium 1 ist.

6. Zusammensetzung zur Verwendung nach einem der Ansprüche 1-5, wobei der Blasenkrebs niedriggradiger oder hochgradiger Blasenkrebs ist.

## Revendications

1. Composition comprenant des liposomes comprenant une fraction de ciblage de folate et un promédicament de mitomycine C à utiliser dans le traitement du cancer de la vessie, dans laquelle le promédicament de mitomycine C est un conjugué de mitomycine C attaché de manière libérable à une fraction lipophile, et est de la forme : 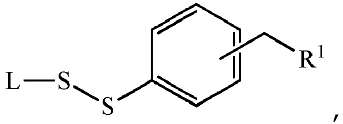 dans laquelle L est la fraction hydrophobe, R¹ représente un résidu de mitomycine C et l'orientation du groupe -CH₂R¹ est sélectionnée parmi la position ortho et la position para.

2. Composition à utiliser selon la revendication 1, dans laquelle la composition est formulée pour une administration par instillation intravésicale.

3. Composition à utiliser selon la revendication 1 ou la revendication 2, dans laquelle la composition est formulée pour retenir les liposomes dans la vessie pendant une période comprise entre environ 15 minutes et environ 2 heures.

4. Composition à utiliser selon l'une quelconque des revendications 1 à 3, dans laquelle la composition est formulée pour une administration avec un agent réducteur afin d'initier la libération du conjugué de promédicament de mitomycine C de la fraction lipophile attachée.

5. Composition à utiliser selon l'une quelconque des revendications 1 à 4, dans laquelle le cancer de la vessie est un cancer de la vessie de stade 0 ou un cancer de la vessie de stade 1.

6. Composition à utiliser selon l'une quelconque des revendications 1 à 5, dans laquelle le cancer de la vessie est un cancer de la vessie de grade faible ou de grade élevé.
